# EUROPEAN PATENT SPECIFICATION

(11) **EP 4 033 983 B1**
(45) Date of publication and mention of the grant of the patent: **28.01.2026**
(21) Application number: 20867251.9
(22) Date of filing: 24.09.2020
(51) Int. Cl.: A61B 8/00, G01S 7/52, G01S 15/89

(54) **METHODS AND APPARATUS FOR CONFIGURING AN ULTRASOUND SYSTEM WITH IMAGING PARAMETER VALUES**
VERFAHREN UND VORRICHTUNG ZUR KONFIGURATION EINES ULTRASCHALLSYSTEMS MIT BILDGEBUNGSPARAMETERWERTEN
PROCÉDÉS ET APPAREILS POUR CONFIGURER UN SYSTÈME À ULTRASONS À L'AIDE DE PLUSIEURS VALEURS DE PARAMÈTRES D'IMAGERIE

(30) Priority: 27.09.2019 US 201962907532 P
(43) Date of publication of application: 03.08.2022
(73) Proprietor: BFLY Operations, Inc., Burlington, MA 01803 (US)
(72) Inventor: GAFNER, Tomer, Forest Hills, NY 11375 (US); SILBERMAN, Nathan, Brooklyn, NY 11201 (US); HOWELL, Audrey, Guilford, CT 06437 (US)
(74) Representative: Osha BWB
(86) International application number: PCT/US2020/052476
(87) International publication number: WO 2021/061971

(56) References cited:
- WO-A1-2019/084411
- WO-A1-2019/084411
- US-A1- 2004 267 124
- US-A1- 2009 099 449
- US-A1- 2013 345 563
- US-A1- 2016 058 426
- US-A1- 2017 156 698
- US-A1- 2018 136 896
- US-B2- 9 743 911

## Description

### FIELD

Generally, the aspects of the technology described herein relate to ultrasound data collection. Some aspects relate to configuring an ultrasound system with imaging parameter values.

### BACKGROUND

Ultrasound systems may be used to perform diagnostic imaging and/or treatment, using sound waves with frequencies that are higher with respect to those audible to humans. Ultrasound imaging may be used to see internal soft tissue body structures, for example to find a source of disease or to exclude any pathology. When pulses of ultrasound are transmitted into tissue (e.g., by using a pulser in an ultrasound imaging device), sound waves are reflected off the tissue, with different tissues reflecting varying degrees of sound. These reflected sound waves may then be recorded and displayed as an ultrasound image to the operator. The strength (amplitude) of the sound signal and the time it takes for the wave to travel through the body provide information used to produce the ultrasound image. Many different types of images can be formed using ultrasound systems, including real-time images. For example, images can be generated that show two-dimensional cross-sections of tissue, blood flow, motion of tissue over time, the location of blood, the presence of specific molecules, the stiffness of tissue, or the anatomy of a three-dimensional region.

US 9 743 911 B2 describes a method and a system for automatic control of image quality in imaging of an object using an ultrasound system. The method comprises transmitting image generating signals into the object using selected system parameter sets of the imaging system. The imaging system has a number of different system parameter sets based on an image ranking measure reflecting a subjective expert opinion of a pre-defined set of images. The captured images are analyzed with respect to at least one image quality feature to determine an image quality metric for each image. The respective image quality metric for each image are analyzed to identify an image associated with a highest image quality metric and the system parameter set used for generating the image associated with the highest image quality metric can be selected as system parameter set for imaging of the object.

### SUMMARY

According to one aspect of the invention, an ultrasound system is provided as defined in claim 1.

According to another aspect of the invention, a method for configuring an ultrasound system to produce a plurality of sets of ultrasound images is provided as defined in claim 12.

Embodiments of the system and the method are defined in the dependent claims.

A further aspect includes a non-transitory computer-readable storage medium as defined in claim 14.

### BRIEF DESCRIPTION OF THE DRAWINGS

Various aspects and embodiments will be described with reference to the following exemplary and non-limiting figures. It should be appreciated that the figures are not necessarily drawn to scale. Items appearing in multiple figures are indicated by the same or a similar reference number in all the figures in which they appear.
FIGs. 1A-1B illustrate an example process for configuring an ultrasound imaging device with imaging parameter values, in accordance with certain embodiments described herein;
FIG. 2 illustrates an example graphical user interface, in accordance with certain embodiments described herein;
FIG. 3 illustrates another example graphical user interface, in accordance with certain embodiments described herein;
FIG. 4 illustrates another example graphical user interface, in accordance with certain embodiments described herein;
FIG. 5 illustrates another example graphical user interface, in accordance with certain embodiments described herein;
FIG. 6 illustrates another example graphical user interface, in accordance with certain embodiments described herein; and
FIG. 7 shows a schematic block diagram illustrating aspects of an example ultrasound system upon which various aspects of the technology described herein may be practiced.

### DETAILED DESCRIPTION

An ultrasound system typically includes preprogrammed parameter values for configuring the ultrasound system to image various anatomical features. For example, a given anatomical feature may be located at a certain depth from the surface of a subject, and the depth may determine imaging parameters such as frequency. Thus, for example, a user wishing to scan a subject's heart may manually select imaging parameter values associated with the heart on the ultrasound imaging system, and this selection may configure the ultrasound system with the preprogrammed parameter values for cardiac ultrasound imaging. The user may, for example, make the selection by choosing a menu option on a display screen or pressing a physical button.

The inventors have recognized that the ease for a user to perform ultrasound imaging may be improved by automatically determining imaging parameter values for imaging a particular region of a subject. In particular, the inventors have recognized that multiple sets of imaging parameter values may be tested to determine which set is most appropriate for imaging a particular region on a subject. Testing the multiple sets of imaging parameter values may include obtaining, from the ultrasound system, multiple sets of ultrasound images (where a set may include one or more ultrasound images) produced from the same location on a subject using different sets of imaging parameter values. Once sets of ultrasound images have been produced using all the sets of imaging parameter values to be tested, which of the imaging parameters values produced the "best" set of ultrasound images may be determined by calculating a quality for each of the sets of ultrasound images. The quality may be calculated, for example, as a confidence that a view classifier recognizes an anatomical region in the set of ultrasound images. The ultrasound system may then be configured to prompt the user for input on whether the ultrasound system should configure itself to use the set of imaging parameter values that produced the "best" set of ultrasound images. This may be helpful in helping a user to not need to access a preset selection menu if the user changes the anatomical region being imaged.

For example, a user may place an ultrasound imaging device included in the ultrasound system at a single location at the subject's heart. The ultrasound system may automatically produce multiple sets of ultrasound images from that one location at the subject's heart using imaging parameter values optimized for the heart, the abdomen, the bladder, or other anatomical features or regions. The ultrasound system may then determine that the imaging parameter values for the heart produced the "best" data, and prompt the user for input on whether the ultrasound system should configure itself to continue imaging using the imaging parameter values for the heart. The user may then select to continue imaging using the imaging parameter values for the heart, and the ultrasound system may configure itself accordingly.

It should be appreciated that the embodiments described herein may be implemented in any of numerous ways. Examples of specific implementations are provided below for illustrative purposes only. It should be appreciated that these embodiments and the features/capabilities provided may be used individually, all together, or in any combination of two or more, as aspects of the technology described herein are not limited in this respect.

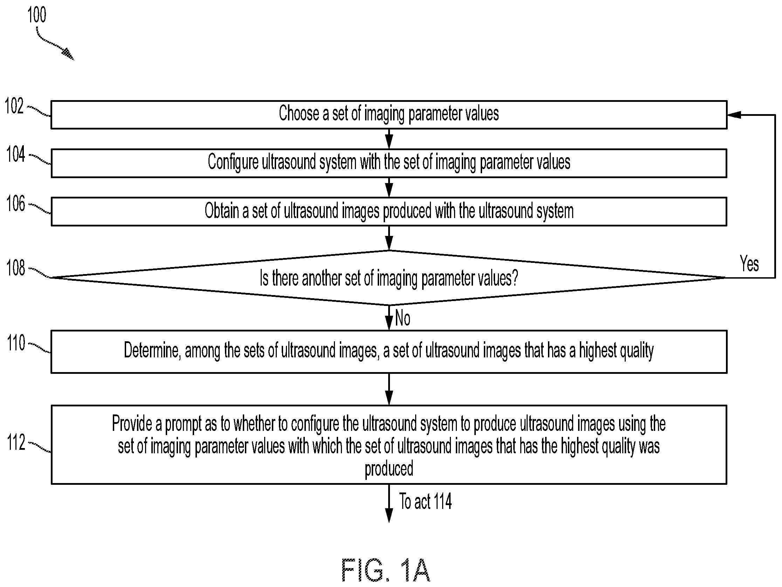

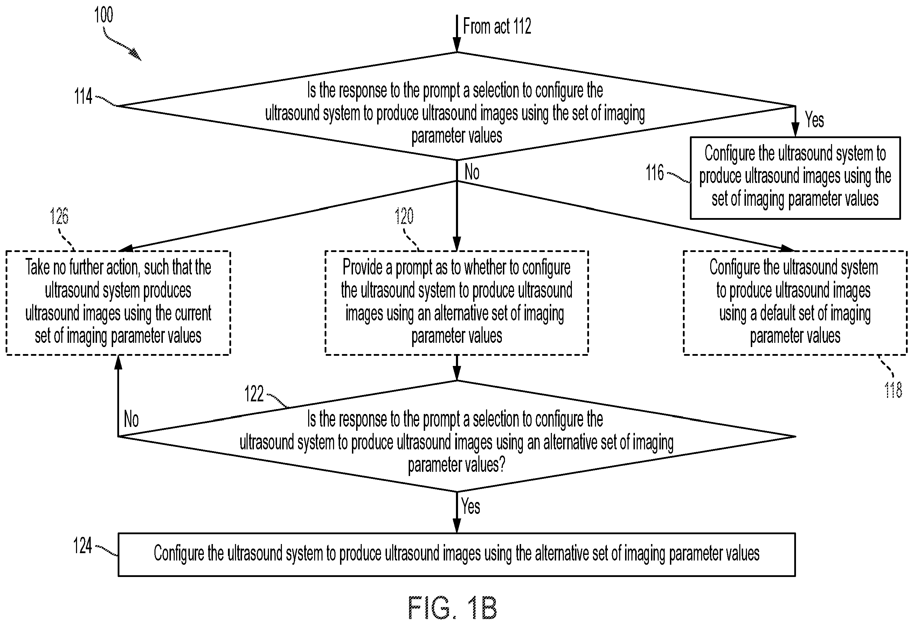

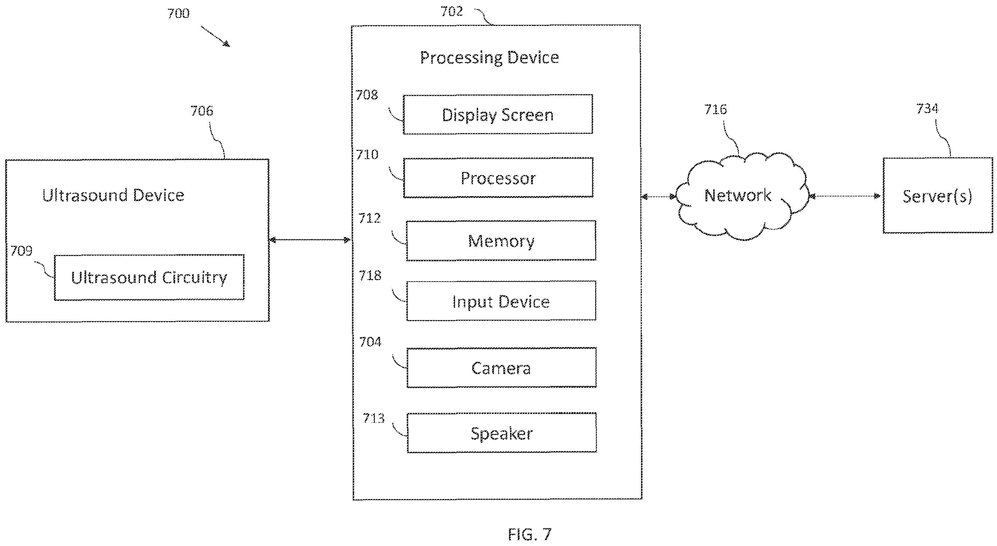

FIGs. 1A-1B illustrate an example process 100 for configuring an ultrasound system with imaging parameter values, in accordance with certain embodiments described herein. The process 100 may be performed by, for example, processing circuitry in the ultrasound system. The ultrasound system may include an ultrasound imaging device used for imaging a subject as well as one or more external devices (e.g., a mobile phone, tablet, laptop, or server) in operative communication with the ultrasound imaging device, and the processing circuitry may be in either or both of these devices. Ultrasound systems and devices are described in more detail with reference to FIGs. 7-9.

Process 100 generally includes searching through and testing multiple sets of imaging parameter values to determine, based on certain criteria, a set that is most appropriate for imaging a particular region on a subject. Testing the multiple sets of imaging parameter values includes obtaining, from the ultrasound system, multiple sets of ultrasound images produced from the same location on a subject using different sets of imaging parameter values (acts 102, 104, 106, and 108). In particular, during each iteration through acts 102, 104, and 106, a different set of ultrasound images is produced using a different set of imaging parameter values. Once sets of ultrasound images have been produced using all the sets of imaging parameter values to be tested, process 100 determines which of the imaging parameter values produced the "best" set of ultrasound images, as determined by calculating a quality for each of the sets of ultrasound images (act 110). In some embodiments, the sets of imaging parameter values to be tested may be any two or more sets of imaging parameter values available to the ultrasound system. Process 100 further includes providing a user with a prompt for input on whether the ultrasound system should configure itself to continue imaging with the imaging parameter values that produced the "best" set of ultrasound images (act 112). For example, a user may place an ultrasound imaging device included in the ultrasound system at a single location at the subject's heart. The ultrasound system may automatically produce multiple sets of ultrasound images from that one location at the subject's heart using imaging parameter values optimized for the heart, the abdomen, the bladder, or other anatomical features or regions. The ultrasound system may then determine that the imaging parameter values for the heart produced the "best" data, and prompt the user for input on whether the ultrasound system should configure itself to continue imaging using the imaging parameter values for the heart. The user may then select to continue imaging using the imaging parameter values for the heart, and the ultrasound system may configure itself accordingly.

In act 102, the processing circuitry chooses values for a set of imaging parameters. The imaging parameters may be parameters governing how the ultrasound system performs ultrasound imaging. Non-limiting examples of imaging parameters that may be included in the set of imaging parameters are frequency, gain, frame rate, power, the speed of sound, and azimuthal/elevational focus.

In some embodiments, the processing circuitry may choose imaging parameter values corresponding to an ultrasound imaging preset. The ultrasound imaging preset may be a predetermined set of imaging parameter values optimized for imaging a particular anatomical region (e.g., cardiac, carotid, abdomen, extremities, bladder, musculoskeletal, uterus, as non-limiting examples). Presets may be further optimized based on the subject (e.g., a pediatric cardiac preset and an adult cardiac preset) and/or based on whether deep or superficial portions of the anatomical region are to be imaged (e.g., a musculoskeletal superficial preset and a musculoskeletal deep preset).

The ultrasound system may be programmed with a group of ultrasound imaging presets corresponding to anatomical regions that the ultrasound imaging device is capable of imaging. Each time the processing circuitry chooses a set of imaging parameter values (as described below, the ultrasound imaging device may iterate through act 102 multiple times), the processing circuitry may retrieve a different preset from the group. In some embodiments, a particular group of preferred ultrasound imaging presets may be associated with a user. For example, a user may choose preferred presets that s/he anticipates using frequently (e.g., if the user is a cardiologist, the user may choose cardiac and carotid presets). As another example, preferred presets may be associated with a user based on the user's past history (e.g., if the user most often uses cardiac and abdominal presets, the cardiac and abdominal presets may be automatically associated with the user). In such embodiments, each time the processing circuitry chooses a set of imaging parameter values, the processing circuitry may retrieve a different preset from the preferred group of presets associated with the user. In some embodiments, a user may input (e.g., by selecting an option from a menu on a graphical user interface, pressing a physical button, using a voice command) a particular ultrasound imaging protocol into the ultrasound system. The ultrasound imaging protocol may require scanning particular anatomical regions, but the order in which the user will scan the particular anatomical regions may not be known. In such embodiments, each time the processing circuitry chooses a set of imaging parameter values, the processing circuitry may retrieve a different preset from a group of presets associated with the anatomical regions that are scanned as part of the ultrasound imaging protocol. For example, a FAST (Fast Assessment with Sonography in Trauma) exam may include scanning the heart and abdomen, and therefore if the user inputs that s/he is performing a FAST exam, each time the processing circuitry chooses a set of imaging parameter values, the ultrasound system may retrieve either a cardiac preset or an abdominal preset. Another example protocol may be the Rapid Ultrasound for Shock and Hypotension (RUSH) exam, which may include collecting various views of the heart, vena cava, Morison's pouch, spleen, kidney, bladder, aorta, and lungs.

In some embodiments, each time the processing circuitry chooses a set of imaging parameter values, the processing circuitry may choose a different value from a portion of all possible values for the imaging parameters, such that after multiple iterations through act 102, the processing circuitry may have iterated through a portion of all combinations of the imaging parameters. For example, in a non-limiting illustrative example in which the only imaging parameter is frequency, if the ultrasound imaging device is capable of imaging at frequencies of 1-15 MHz, the processing circuitry may choose a different one of 1 MHz, 2 MHz, 3 MHz, 4 MHz, 5 MHz, 6 MHz, 7 MHz, 8 MHz, 9 MHz, 10 MHz, 11 MHz, 12 MHz, 13 MHz, 14 MHz, and 15 MHz during each iteration through act 102. In examples in which the processing circuitry chooses values for multiple imaging parameters (e.g., two or more of frequency, gain, frame rate, and power), the processing circuitry may choose a different combination of the imaging parameters during each iteration through act 102. In other words, the processing circuitry may iterate through a portion of the entire imaging parameter space after multiple iterations through act 102. In general, regardless of how the particular imaging parameter values are chosen, the set of imaging parameter values chosen at act 102 may be different than any other set of imaging parameter values chosen at previous iterations through act 102. The process 100 may then continue to act 104.

In act 104, the processing circuitry configures the ultrasound system with the set of imaging parameter values chosen in act 102. In some embodiments, the processing circuitry may configure the ultrasound system with values for imaging parameters related to ultrasound transmission (e.g., the frequency of ultrasound waves transmitted by the ultrasound system into a subject). In some embodiments, the processing circuitry may configure the ultrasound system with values for imaging parameters related to ultrasound image generation (e.g., the speed of sound within the portion of the subject being imaged, azimuthal/elevational focus, etc.). In some embodiments, a processing device in operative communication with the ultrasound imaging device may transmit an instruction/instructions to the ultrasound imaging device to trigger configuration of the ultrasound imaging device with the imaging parameter values chosen in act 102. This may be helpful when the ultrasound imaging device must be configured with an image parameter value related to transmission of ultrasound waves from the ultrasound imaging device. The process 100 may then proceed to act 106.

In act 106, the processing circuitry obtains a set of ultrasound images (i.e., one or more ultrasound images) produced by the ultrasound system. The set of ultrasound images may be images produced with the ultrasound system as configured (in act 104) with the imaging parameter values chosen in act 102 and may be obtained from the same region of interest on the subject as data produced during a previous iteration through act 106. In embodiments in which the imaging parameters relate to ultrasound transmission, the set of ultrasound images may be produced by transmitting ultrasound waves corresponding to the set of imaging parameter values into the subject and generating the set of ultrasound images from the reflected ultrasound waves. In embodiments in which the imaging parameters relate to image generation, the set of ultrasound images may be produced from reflected ultrasound waves by using the image generation parameter values. In some embodiments, after an ultrasound imaging device has received a set of ultrasound data, the ultrasound imaging device may transmit the set of ultrasound data to a processing device in operative communication with the ultrasound imaging device, and the processing device may generate a set of ultrasound images from the set of ultrasound data. Transmission may occur over a wired communication link (e.g., over Ethernet, a Universal Serial Bus (USB) cable or a Lightning cable) or over a wireless communication link (e.g., over a BLUETOOTH, WiFi, or ZIGBEE wireless communication link). The process 100 may then proceed to act 108.

In act 108, the processing circuitry determines if there is another set of imaging parameter values to test. If there is another set of imaging parameter values to test, the process 100 may proceed to act 102, in which another set of imaging parameter values will be chosen (act 102). Following act 102, the new set of imaging parameter values will be used to configure the ultrasound system (act 104) for producing a set of ultrasound images (act 106). If there is not another set of imaging parameter values to test, the process 100 may proceed to act 110.

Accordingly, with each iteration through acts 102, 104, and 106, a different set of ultrasound images may be obtained using a different set of imaging parameter values, producing multiple sets of ultrasound images after multiple iterations through acts 102, 104, and 106. In embodiments in which different sets of imaging parameter values related to ultrasound transmission are used, the different sets of ultrasound images may be produced by transmitting different ultrasound waves (e.g., having different frequencies) into the subject and generating different images for each set of reflected ultrasound waves. In embodiments in which different sets of imaging parameters values related to image generation are used, the different sets of ultrasound images may be produced by using different image generation parameter values to produce different ultrasound images from the same set of ultrasound waves reflected after transmitting the same set of ultrasound waves. The multiple sets of ultrasound images may be considered test data for testing which set of imaging parameter values a user should be prompted with for continued imaging. As will be described below with reference to act 110, this testing is performed by determining, from among all the sets of ultrasound images produced during multiple iterations through acts 102, 104, and 106, which set of ultrasound images has the highest quality.

In some embodiments, the set of imaging parameters tested may include the frequency of transmitted ultrasound waves. Because the frequency of transmitted ultrasound waves may determine how well anatomical structures at a particular depth can be imaged, determining what frequency produces ultrasound images having the highest quality may help to improve the quality of imaging of anatomical structures at the region of interest.

In some embodiments, the set of imaging parameters tested may include the speed of sound within the subject. Because the speed of sound within a subject may vary depending on how much fat is at the region of interest and the types of organs/tissues at the region of interest, and because the speed of sound affects generation of ultrasound images from reflected ultrasound waves, determining what speed of sound value produces ultrasound images having the highest quality may help to improve the quality of imaging of particular individuals (e.g., those that have more fat and those that have less fat) or particular anatomical structures at the region of interest.

In some embodiments, the set of imaging parameters tested may include the azimuthal and/or elevational focus. Because the azimuthal and/or elevational focus may determine what anatomical structures are in focus in a generated image, determining what azimuthal/elevational focus produces ultrasound images having the highest quality may help to improve the quality of imaging of particular anatomical structures at the region of interest.

In act 110, the processing circuitry may determine among the sets of ultrasound images produced from iterations through acts 102, 104, and 106, a set of ultrasound images that has a highest quality. For example, the processing circuitry may calculate a value for the quality of each particular set of ultrasound images, and associate the quality value with the particular set of imaging parameter values used to produce the particular set of ultrasound images in one or more data structures. For example, quality values may be associated with corresponding imaging parameter values in one data structure, or values for the quality metric may be associated with sets of ultrasound images in one data structure and the sets of ultrasound images may be associated with the corresponding imaging parameter values in another data structure. The processing circuitry may apply any maximum-finding algorithm to such a data structure/data structures in order to find the imaging parameter values that produced the set of ultrasound images having the highest quality. It should be appreciated that depending on the quality metric used, in some embodiments, lower values for the quality metric may be indicative of a higher quality for the ultrasound images (e.g., if the quality metric is a metric of how much noise is in the ultrasound images). In such embodiments, the processing circuitry may determine the set of ultrasound images having the lowest value for the quality value. In some embodiments, if multiple sets of parameters provide sets of ultrasound images having substantially the same quality, one of the sets of parameters may be chosen arbitrarily.

According to the invention, determining the quality of a set of ultrasound images includes determining a confidence that a view classifier recognizes an anatomical region in the set of ultrasound images. In particular, the view classifier may include one or more convolutional neural networks trained to accept a set of ultrasound images (e.g., one or more ultrasound images) as an input and to recognize an anatomical region in the set of ultrasound images. Furthermore, the one or more convolutional neural networks may output a confidence (e.g., between 0% and 100%) in its classification of the anatomical region. The classification may include, for example, recognizing whether an anatomical region in an image represents an apical four chamber or apical two chamber view of the heart. To train the one or more convolutional neural networks to perform classification on images, the one or more convolutional neural networks may be trained with images that have been manually classified. For further description of convolutional neural networks and deep learning techniques, see the description with reference to FIG. 9. A high confidence that an anatomical region has been recognized may be indicative that the imaging parameter values used to produce the set of ultrasound images can be used to produce ultrasound images containing identifiable anatomical structures. Accordingly, a high confidence that an anatomical region has been recognized may correspond to a higher quality image.

In some embodiments, determining the quality of a set of ultrasound images may include determining an image sharpness metric. For example, determining the image sharpness metric for an ultrasound image may include calculating a two-dimensional Fourier transform of the ultrasound image, determining the centroid of the Fourier transformed image, and determining the maximum/minimum/mean/median/sum of the two frequencies at the centroid. A higher value for this metric may correspond to a higher quality image. Determining the image sharpness metric in this way may be more effective after a non-coherent compounding process configured to reduce speckle has been performed.

In some embodiments, determining the quality of a set of ultrasound images may include determining a pixel variation metric. For example, determining the pixel variation metric for an ultrasound image may include dividing an ultrasound image into blocks of pixels, finding the maximum pixel value within each block of pixels, determining the standard deviation of all the pixels in each block of pixels from the maximum pixel value within the block of pixels, and determining the maximum/minimum/mean/median/sum of all the standard deviations across all the blocks of pixels in the image. A lower value for this metric may correspond to a higher quality image.

In some embodiments, determining the quality of a set of ultrasound images may include determining a noise metric. For example, determining the noise metric for an ultrasound image may include using the CLEAN algorithm to find noise components within each pixel of the ultrasound image and determining the maximum, minimum, mean, median and/or sum of the noise components within each pixel of the ultrasound image. A lower value for this metric may correspond to a higher quality image.

In some embodiments, determining the quality of a set of ultrasound images may include determining a total variation metric for the image. For further description of the total variation metric, see Rudin, Leonid I., Stanley Osher, and Emad Fatemi. "Nonlinear total variation based noise removal algorithms." Physica D: nonlinear phenomena 60.1-4 (1992): 259-268.

In some embodiments, determining the quality of a set of ultrasound images may include determining a pixel intensity metric. For example, determining the pixel intensity metric for an ultrasound image may include summing the absolute value/square/any power of the pixel intensities of the ultrasound image. A higher value for this metric may correspond to a higher quality image.

Further description of metrics for determining the quality of an image may be found in Kragh, Thomas J., and A. Alaa Kharbouch, "Monotonic iterative algorithm for minimum-entropy autofocus," Adaptive Sensor Array Processing (ASAP) Workshop, (June 2006), Vol. 53, 2006; and Fienup, J. R., and J. J. Miller, "Aberration correction by maximizing generalized sharpness metrics," JOSA A 20.4 (2003): 609-620.

In some embodiments, one or more of the above metrics may be used in combination with the highest confidence that the view classifier recognizes an anatomical region in the set of ultrasound images, to determine the set of ultrasound images having the highest quality. For example, the sum/mean/median of two or more metrics may be used to determine the set of ultrasound images having the highest quality.

In some embodiments, the processing circuitry may exclude portions of the set of ultrasound images that show reverberation or shadowing prior to determining the quality of a set of ultrasound images. A convolutional neural network may be trained to recognize reverberation or shadowing in portions of ultrasound images. In particular, the training data for the convolutional neural network may include portions of ultrasound images labeled with whether they exhibit reverberation, shadowing, or neither.

In act 112, the processing circuitry provides a prompt as to whether the processing circuitry should configure the ultrasound system to produce ultrasound images using the set of imaging parameter values with which the set of ultrasound images that has the highest quality was produced (i.e., the set of parameters determined in act 110). The prompt may include a notification of the set of imaging parameters, such as a notification of the anatomical region for which the set of imaging parameters is optimized. The prompt may include text displayed on a display screen and/or audio outputted by a speaker. The process 100 proceeds from act 112 to act 114.

In act 114, the processing circuitry receives from the user a response to the prompt provided in act 112. For example, the user may provide the response to the prompt by selecting an option from a display screen and/or speaking a response to the prompt. The response may include, for example, selecting an option to configure the ultrasound system with the set of imaging parameter values determined in act 110 or to not configure the ultrasound system with the set of imaging parameter values. If the processing circuitry receives a response that is a selection to configure the ultrasound system with the set of imaging parameter values, the process proceeds to act 116. If the processing circuitry receives a response that is a selection to not configure the ultrasound system with the imaging parameter values, in some embodiments the process 100 proceeds to act 118, in some embodiments the process 100 proceeds to act 120, and in some embodiments the process 100 proceeds to act 126.

In act 116, the processing circuitry configures the ultrasound system to produce ultrasound images using the set of imaging parameter values determined in act 110. For example, the processing circuitry may transmit an instruction/instructions to the ultrasound imaging device to trigger configuration of the ultrasound imaging device with the imaging parameter values. These imaging parameter values may be used for continued imaging of the region of interest.

As described above, if in act 114, the processing circuitry receives a response that is a selection to not configure the ultrasound system with the imaging parameter values, in some embodiments the process 100 proceeds to act 118, in some embodiments the process 100 proceeds to act 120, and in some embodiments the process 100 proceeds to act 126. In act 118, the processing circuitry configures the ultrasound system to produce ultrasound images using a default set of imaging parameter values (e.g., a set of imaging parameters optimized for imaging the abdomen). For example, the processing circuitry may transmit an instruction/instructions to the ultrasound imaging device to trigger configuration of the ultrasound imaging device with the default set of imaging parameter values.

In act 120, the processing circuitry provides a prompt as to whether to configure the ultrasound system to produce ultrasound images using an alternative set of imaging parameter values. In some embodiments, the alternative set of imaging parameter values may be the set of imaging parameter values with which the set of ultrasound images that has the second-highest quality was produced. In some embodiments, the alternative set of imaging parameter values may be a default set of imaging parameters (e.g., a set of imaging parameters optimized for imaging the abdomen). In some embodiments, the alternative set of imaging parameter values may be a user-selected set of imaging parameters. The process 100 proceeds from act 120 to act 122.

In act 122, the processing circuitry receives from the user a response to the prompt provided in act 120. For example, the user may provide the response to the prompt by selecting an option from a display screen and/or speaking a response to the prompt. If the processing circuitry receives a response that is a selection to configure the ultrasound system with the alternative set of imaging parameter values, the process proceeds to act 124, in which the processing circuitry configures the ultrasound system to produce ultrasound images using the alternative set of imaging parameter values. For example, the processing circuitry may transmit an instruction/instructions to the ultrasound imaging device to trigger configuration of the ultrasound imaging device with the alternative set of imaging parameter values. If, in act 122, the processing circuitry receives a response that is a selection to not configure the ultrasound system with the alternative set of imaging parameter values, the process proceeds to act 126. In act 126, the processing device takes no action, such that the ultrasound system may continue to use the set of imaging parameters with which it is currently configured.

In some embodiments, the process 100 may automatically proceed periodically. In other words, every time a set period of time elapses, the process 100 may automatically proceed in order to determine which set of imaging parameter values should be suggested to the user for imaging during the next period of time. In other embodiments, the process 100 may automatically proceed based on the processing circuitry detecting that the ultrasound system has begun imaging the subject after not imaging the subject for a threshold period of time. In some embodiments, determining that the ultrasound system is not imaging a subject may include determining that the sum/mean/median of pixel values in a produced ultrasound image does not exceed a threshold value, and determining that the ultrasound system is imaging a subject may include determining that the sum/mean/median of pixel values in a produced ultrasound image does exceed a threshold value. In some embodiments, a convolutional neural network may be trained to recognize whether an ultrasound image was collected when an ultrasound imaging device was imaging a subject. The training data for the convolutional neural network may include ultrasound images labeled with whether the ultrasound image was collected when the ultrasound imaging device was imaging a subject or not. In some embodiments, determining whether the ultrasound system is imaging a subject may include calculating a cross-correlation between an ultrasound image collected by the ultrasound imaging device and a calibrated ultrasound image collected when there is an interface between an ultrasound imaging device and air. A cross-correlation having a mean to peak ratio that exceeds a threshold value (e.g., the peak cross-correlation value is over 20 times the mean cross-correlation value) may indicate that the ultrasound imaging device is not imaging a subject. In some embodiments, determining whether the ultrasound system is imaging a subject may include analyzing (e.g., using a fast Fourier transform) whether a period of intensities across an ultrasound image or across A-lines is highly correlated (e.g., the peak cross-correlation value is over 20 times the mean cross-correlation value), which may be indicative of reverberations and that there is an interface between the ultrasound imaging device and air. In some embodiments, determining whether the ultrasound system is imaging a subject may include calculating a cross-correlation over vertical components, such as columns of an image (or a subset of an image's columns and/or a subset of the pixels of columns of the image) collected perpendicular to the probe face or A-lines collected perpendicular to the probe face. If the ultrasound system is not imaging a subject, a mean to peak ratio of the cross-correlation may be over a specified threshold (e.g., the peak cross-correlation value may be over 20 times the mean cross-correlation value).

Detecting that the ultrasound system has begun imaging the subject after not imaging the subject for a threshold period of time may correspond to detecting the beginning of a new imaging session. Determining a best set of imaging parameter at the beginning of an imaging session, but not during an imaging session, may be helpful for conserving power expended in producing multiple sets of ultrasound images and calculating values for a quality metric for each set of ultrasound images. Such embodiments may be appropriate in cases in which the region of a subject being scanned may not change in a way that would require substantial changes to imaging parameter values. For example, an imaging session including just imaging of the cardiac area may not require substantial changes to imaging parameter values during the imaging session. To conserve power while detecting whether the ultrasound system has begun imaging the subject after not imaging the subject for a threshold period of time, in some embodiments, detecting that the ultrasound system has begun imaging the subject may include configuring the ultrasound system with a set of imaging parameter values that use less power than the sets of imaging parameter values in act 104. (As referred to herein, a set of imaging parameter values that uses a certain amount or degree of power should be understood to mean that the ultrasound system uses the amount or degree of power when configured with the set of imaging parameter values). This may be a means of conserving power, as the ultrasound system may use lower power to collect ultrasound images of low but sufficient quality to detect that the ultrasound system has begun imaging a subject. Once this detection has occurred, the ultrasound system may use higher power to collect ultrasound images having higher quality sufficient for clinical use. The set of imaging parameter values that enables the ultrasound system to collect ultrasound image at lower power may include, for example, a lower pulse repetition frequency (PRF), lower frame rate, shorter receive interval, reduced number of transmits per image, and lower pulser voltage.

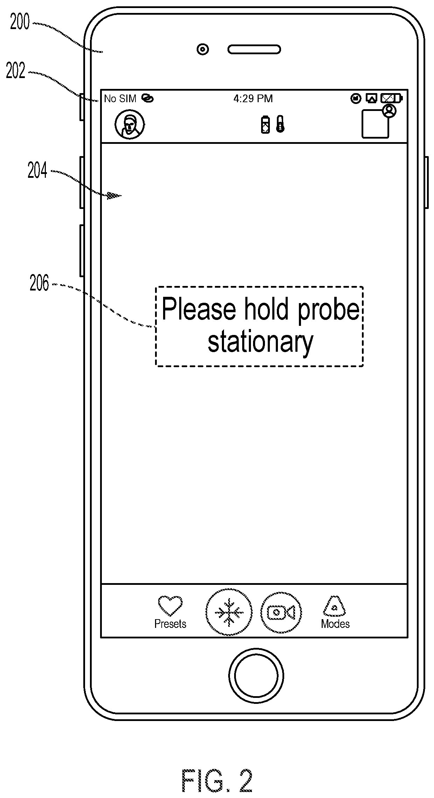

In some embodiments, until the processing circuitry has configured the ultrasound system to produce ultrasound images using the set of imaging parameter values with which the set of ultrasound images that has the highest quality was produced (i.e., until act 112 has been completed), the processing circuitry may generate a notification to hold the ultrasound imaging device substantially stationary (see, e.g., FIG. 2). This may be helpful in ensuring that all the imaging parameter values are evaluated for how appropriate they are for use at the particular region of interest. In some embodiments, the notification may be graphically displayed on a display of the processing device in operative communication with the ultrasound imaging device. In some embodiments, the notification may be played audibly by speakers of the processing device in operative communication with the ultrasound imaging device.

It should be appreciated that while the above description of process 100 references sets of ultrasound images (e.g., collecting sets of ultrasound images, calculating the quality of sets of ultrasound images, inputting sets of ultrasound images to neural networks, etc.) the process 100 may also be applied to other types of ultrasound data (e.g., raw acoustical data, multilines, spatial coordinates, or other types of data generated from raw acoustical data).

FIGs. 2-6 illustrate example graphical user interfaces (GUIs) 204, 304, 404, 504, and 604, respectively, that may be generated by a processing device 200 in operative communication with an ultrasound imaging device, and displayed by a display 202 of the processing device 200, in accordance with certain embodiments described herein.

FIG. 2 illustrates the GUI 204. As described above, it may be helpful to generate a notification to hold the ultrasound imaging device substantially stationary until an ultrasound system has been configured to produce ultrasound images using a set of imaging parameter values with which a set of ultrasound images that has the highest quality was produced. The GUI 204 shows a graphical notification 206 to hold the ultrasound imaging device stationary. It should be appreciated that the exact form and text of the notification 206 is not limiting, and other forms and texts for the notification 206 that convey the similar intent may be used.

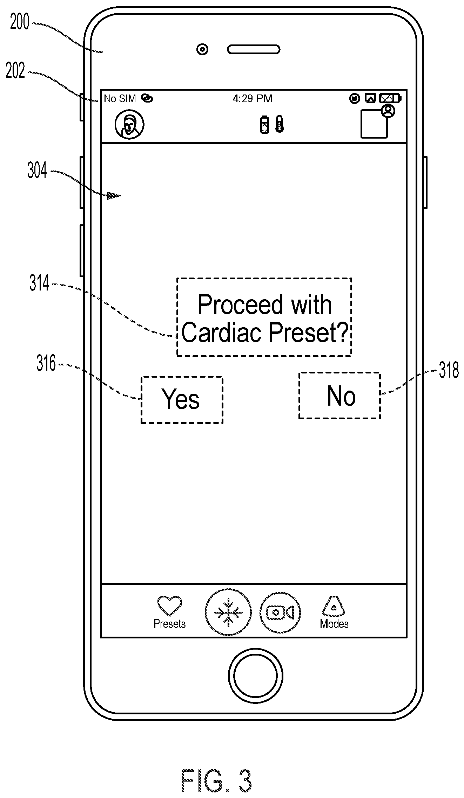

FIG. 3 illustrates the GUI 304. The GUI 304 shows a prompt 314 that includes a notification of a preset (in the example of FIG. 3, a cardiac preset) and a prompt as to whether to configure the ultrasound system with this preset. The preset in the prompt 314 may be the set of imaging parameter values determined to produce ultrasound images having a highest quality (as described with reference to act 110). The GUI 304 further includes a yes option 316 and a no option 318. In response to receiving a selection of the yes option 316, the ultrasound system may be configured with the cardiac preset. In response to receiving a selection of the no option 318, in some embodiments the ultrasound system may be configured with a default preset, or the ultrasound system may continue to use the preset with which it is currently configured, or a prompt may be provided as to whether the ultrasound system should be configured with the preset that produced the second-highest quality ultrasound images. It should be appreciated that while the example prompt 314 prompts a user whether to configure the ultrasound system with a cardiac preset, the prompt 314 may prompt the user whether to configure the ultrasound system with any preset. It should also be appreciated that the exact forms and texts of the prompt 314, the yes option 316, and the no option 318 are not limiting, and other forms and texts may be used.

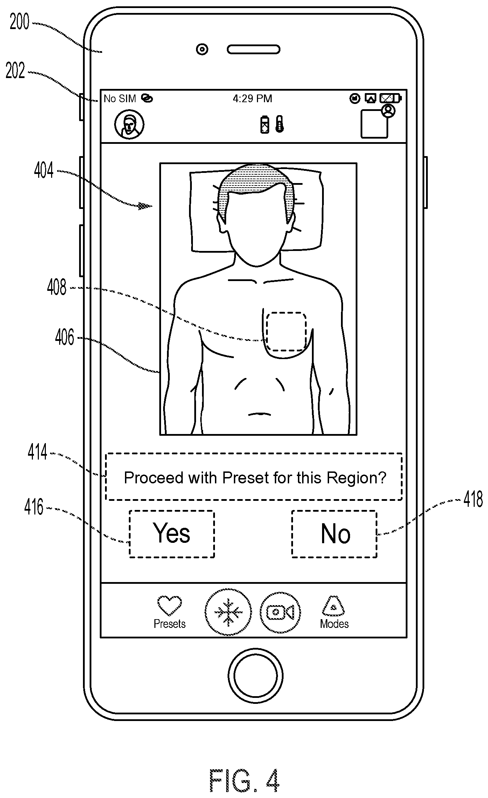

FIGs. 4-6 show example graphical user interfaces that may be useful, for example, in imaging protocols (e.g., FAST and RUSH) that include imaging multiple anatomic regions and may benefit from efficient automatic selection and changing of optimal imaging parameters depending on the anatomic region currently being imaged. FIG. 4 illustrates the GUI 404. The GUI 404 shows an image of a subject 406 and an indicator 408. The indicator 408 indicates on the image of the subject 406 an anatomical region corresponding to the preset that produced the highest quality set of images (as described with reference to act 110). In the example of FIG. 4, the indicator 408 indicates that a cardiac preset has been chosen. The GUI 404 further shows a prompt 414 as to whether to configure the ultrasound system with the preset indicated by the indicator 408. The GUI 404 further includes a yes option 416 and a no option 418. In response to receiving a selection of the yes option 416, the ultrasound system may be configured with the cardiac preset. In response to receiving a selection of the no option 418, in some embodiments the ultrasound system may be configured with a default preset, or the ultrasound system may continue to use the preset with which it is currently configured, or a prompt may be provided as to whether the ultrasound system should be configured with the preset that produced the second-highest quality ultrasound images. It should be appreciated that while the example indicator 408 indicates the cardiac region, the indicator 408 may indicate any anatomical region. It should also be appreciated that the exact forms of the image of the subject 406 and the indicator 408 are not limiting, and other forms of the image of the subject 406 and the indicator 408 may be used. It should also be appreciated that the exact forms and texts of the prompt 414, the yes option 416, and the no option 418 are not limiting, and other forms and texts may be used. In some embodiments, the user may optionally change the preset selected by, for example, tapping another anatomical region on the image of the subject 406 on the GUI 404.

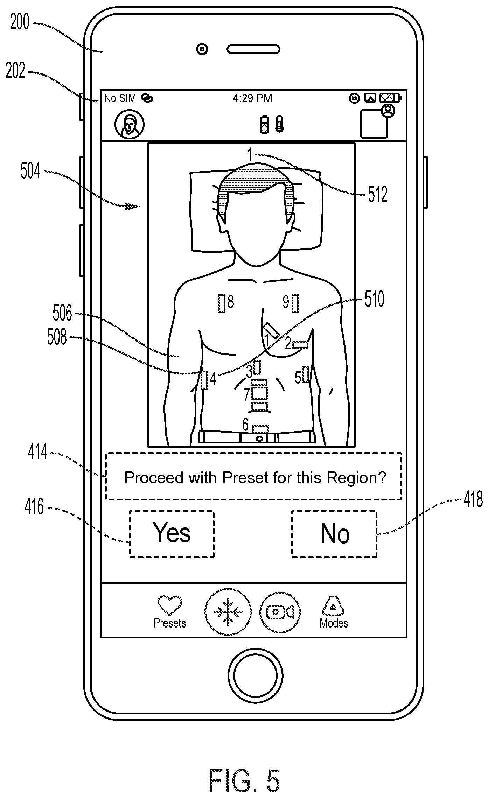

FIG. 5 shows a non-limiting alternative to the GUI 404 of FIG. 4. While the GUI 404 indicates a preset with the indicator 408, the GUI 504 of FIG. 5 indicates a preset with a number 512. The GUI 504 shows an image of a subject 706 and indications 508 of anatomical regions that are scanned as part of an imaging protocol. In the example of FIG. 5, the GUI 504 shows nine regions that are scanned as part of the RUSH protocol. The GUI 504 further shows numbers 510, each corresponding to one of the anatomical regions that is scanned as part of the imaging protocol. Additionally, the GUI 504 shows the number 512 at the top of the GUI 504. The number 512 matches one of the numbers 510 and thereby indicates which of the anatomical regions corresponds to the preset that produced the highest quality set of images (as described with reference to act 110). The GUI 504 further shows the prompt 414 as to whether to configure the ultrasound system with the preset indicated by the number 512, the yes option 416, and the no option 418. It should be appreciated that while the number 512 in FIG. 5 indicates the cardiac region, the number 512 may indicate any anatomical region. Additionally, while the indications 508 of anatomical regions correspond to anatomical regions that may be scanned as part of the RUSH protocol, the indications 508 of anatomical regions may correspond to other imaging protocols. It should also be appreciated that the exact forms of the image of the subject 506, the indications 508 of anatomical regions, the numbers 510, and the number 512 are not limiting, and other forms of the image of the subject 506, the indications 508 of anatomical regions, the numbers 510, and the number 512. For example, the number 512 may be displayed in another region of the GUI 504. In some embodiments, if the user wishes to change the preset selected, the user may tap another anatomical region, indication 508, and/or number 510 on the GUI 504.

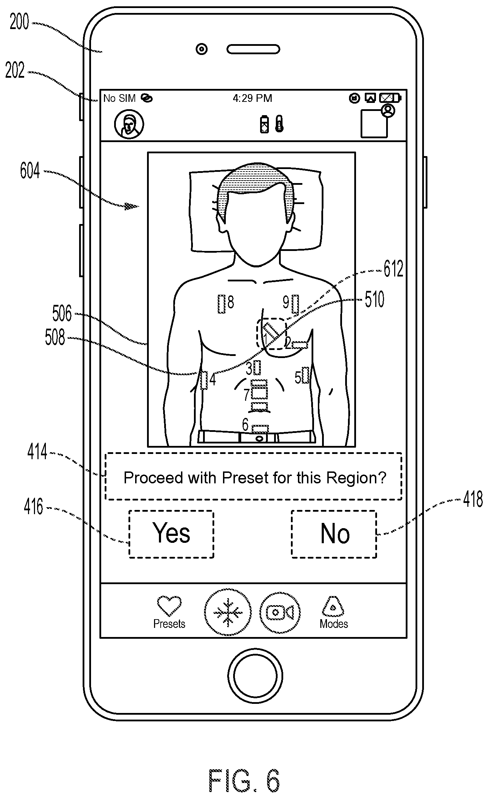

FIG. 6 shows another non-limiting alternative to the GUIs 404 and 504 of FIGs. 4 and 5, respectively. While the GUIs 404 and 504 indicate the selected preset with the indicator 408 and the number 512, respectively, the GUI 604 of FIG. 6 indicates the selected preset with an indicator 612. The indicator 612 highlights the anatomical region that corresponds to the preset that produced the highest quality set of images (as described with reference to act 110). In the example of FIG. 6, the indicator 612 encircles one of the indications 508 and one of the numbers 510. The GUI 604 further shows the prompt 414 as to whether to configure the ultrasound system with the preset indicated by the indicator 612, the yes option 416, and the no option 418. It should be appreciated that other manners for highlighting an anatomical region are possible, such as changing the color of the indication 508 and/or the number 510.

Various inventive concepts may be embodied as one or more processes, of which examples have been provided. The acts performed as part of each process may be ordered in any suitable way. Thus, embodiments may be constructed in which acts are performed in an order different than illustrated, which may include performing some acts simultaneously, even though shown as sequential acts in illustrative embodiments. Further, one or more of the processes may be combined and/or omitted, and one or more of the processes may include additional steps.

FIG. 7 illustrates a schematic block diagram of an example ultrasound system 700 upon which various aspects of the technology described herein may be practiced. The ultrasound system 700 includes an ultrasound device 706, a processing device 702, a network 716, and one or more servers 734.

The ultrasound device 706 includes ultrasound circuitry 709. The processing device 702 includes a camera 704, a display screen 708, a processor 710, a memory 712, an input device 718, and a speaker 713. The processing device 702 is in wired (e.g., through a lightning connector or a mini-USB connector) and/or wireless communication (e.g., using BLUETOOTH, ZIGBEE, and/or WiFi wireless protocols) with the ultrasound device 706. The processing device 702 is in wireless communication with the one or more servers 734 over the network 716. However, the wireless communication with the server 734 is optional.

The ultrasound device 706 may be configured to generate ultrasound data that may be employed to generate an ultrasound image. The ultrasound device 706 may be constructed in any of a variety of ways. In some embodiments, the ultrasound device 706 includes a transmitter that transmits a signal to a transmit beamformer which in turn drives transducer elements within a transducer array to emit pulsed ultrasonic signals into a structure, such as a patient. The pulsed ultrasonic signals may be back-scattered from structures in the body, such as blood cells or muscular tissue, to produce echoes that return to the transducer elements. These echoes may then be converted into electrical signals by the transducer elements and the electrical signals are received by a receiver. The electrical signals representing the received echoes are sent to a receive beamformer that outputs ultrasound data. The ultrasound circuitry 709 may be configured to generate the ultrasound data. The ultrasound circuitry 709 may include one or more ultrasonic transducers monolithically integrated onto a single semiconductor die. The ultrasonic transducers may include, for example, one or more capacitive micromachined ultrasonic transducers (CMUTs), one or more CMOS (complementary metal-oxide-semiconductor) ultrasonic transducers (CUTs), one or more piezoelectric micromachined ultrasonic transducers (PMUTs), and/or one or more other suitable ultrasonic transducer cells. In some embodiments, the ultrasonic transducers may be formed the same chip as other electronic components in the ultrasound circuitry 709 (e.g., transmit circuitry, receive circuitry, control circuitry, power management circuitry, and processing circuitry) to form a monolithic ultrasound device. The ultrasound device 706 may transmit ultrasound data and/or ultrasound images to the processing device 702 over a wired (e.g., through a lightning connector or a mini-USB connector) and/or wireless (e.g., using BLUETOOTH, ZIGBEE, and/or WiFi wireless protocols) communication link.

Referring now to the processing device 702, the processor 710 may include specially-programmed and/or special-purpose hardware such as an application-specific integrated circuit (ASIC). For example, the processor 710 may include one or more graphics processing units (GPUs) and/or one or more tensor processing units (TPUs). TPUs may be ASICs specifically designed for machine learning (e.g., deep learning). The TPUs may be employed to, for example, accelerate the inference phase of a neural network. The processing device 702 may be configured to process the ultrasound data received from the ultrasound device 706 to generate ultrasound images for display on the display screen 708. The processing may be performed by, for example, the processor 710. The processor 710 may also be adapted to control the acquisition of ultrasound data with the ultrasound device 706. The ultrasound data may be processed in real-time during a scanning session as the echo signals are received. In some embodiments, the displayed ultrasound image may be updated a rate of at least 5Hz, at least 10 Hz, at least 20Hz, at a rate between 5 and 60 Hz, at a rate of more than 20 Hz. For example, ultrasound data may be acquired even as images are being generated based on previously acquired data and while a live ultrasound image is being displayed. As additional ultrasound data is acquired, additional frames or images generated from more-recently acquired ultrasound data are sequentially displayed. Additionally, or alternatively, the ultrasound data may be stored temporarily in a buffer during a scanning session and processed in less than real-time.

The processing device 702 may be configured to perform certain of the processes (e.g., the process 10) described herein using the processor 710 (e.g., one or more computer hardware processors) and one or more articles of manufacture that include non-transitory computer-readable storage media such as the memory 712. The processor 710 may control writing data to and reading data from the memory 712 in any suitable manner. To perform certain of the processes described herein, the processor 710 may execute one or more processor-executable instructions stored in one or more non-transitory computer-readable storage media (e.g., the memory 712), which may serve as non-transitory computer-readable storage media storing processor-executable instructions for execution by the processor 710. The camera 704 may be configured to detect light (e.g., visible light) to form an image. The camera 704 may be on the same face of the processing device 702 as the display screen 708. The display screen 708 may be configured to display images and/or videos, and may be, for example, a liquid crystal display (LCD), a plasma display, and/or an organic light emitting diode (OLED) display on the processing device 702. The input device 718 may include one or more devices capable of receiving input from a user and transmitting the input to the processor 710. For example, the input device 718 may include a keyboard, a mouse, a microphone, touch-enabled sensors on the display screen 708, and/or a microphone. The display screen 708, the input device 718, the camera 704, and the speaker 713 may be communicatively coupled to the processor 710 and/or under the control of the processor 710.

It should be appreciated that the processing device 702 may be implemented in any of a variety of ways. For example, the processing device 702 may be implemented as a handheld device such as a mobile smartphone or a tablet. Thereby, a user of the ultrasound device 706 may be able to operate the ultrasound device 706 with one hand and hold the processing device 702 with another hand. In other examples, the processing device 702 may be implemented as a portable device that is not a handheld device, such as a laptop. In yet other examples, the processing device 702 may be implemented as a stationary device such as a desktop computer. The processing device 702 may be connected to the network 716 over a wired connection (e.g., via an Ethernet cable) and/or a wireless connection (e.g., over a WiFi network). The processing device 702 may thereby communicate with (e.g., transmit data to) the one or more servers 734 over the network 716. For further description of ultrasound devices and systems, see U.S. Patent Application No. 15/415,434 titled "UNIVERSAL ULTRASOUND DEVICE AND RELATED APPARATUS AND METHODS," filed on January 25, 2017 and published as U.S. Pat. App. Publication No. 2017-0360397 A1 (and assigned to the assignee of the instant specification).

FIG. 7 should be understood to be non-limiting. For example, the ultrasound system 700 may include fewer or more components than shown and the processing device 702 and ultrasound device 706 may include fewer or more components than shown.

Various aspects of the present disclosure may be used alone, in combination, or in a variety of arrangements not specifically discussed in the embodiments described in the foregoing and is therefore not limited in its application to the details and arrangement of components set forth in the foregoing description or illustrated in the drawings. For example, aspects described in one embodiment may be combined in any manner with aspects described in other embodiments.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

Use of ordinal terms such as "first," "second," "third," etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements.

The terms "approximately" and "about" may be used to mean within ±20% of a target value in some embodiments, within ±10% of a target value in some embodiments, within ±5% of a target value in some embodiments, and yet within ±2% of a target value in some embodiments. The terms "approximately" and "about" may include the target value.

Also, the phraseology and terminology used herein is for the purpose of description and should not be regarded as limiting. The use of "including," "comprising," or "having," "containing," "involving," and variations thereof herein, is meant to encompass the items listed thereafter and equivalents thereof as well as additional items.

Having described above several aspects of at least one embodiment, it is to be appreciated various alterations, modifications, and improvements will readily occur to those skilled in the art. Accordingly, the foregoing description and drawings are by way of example only.

## Claims

1. An ultrasound system (700) comprising:
an ultrasound imaging device (706); and
a processing device (702; 200) that:
configures the ultrasound system (700) to produce a plurality of sets of ultrasound images by: selecting, for a subsequent obtaining of each respective set of the plurality of sets of ultrasound images, a different respective set of a plurality of sets of imaging parameter values;
obtains, using the ultrasound imaging device, and based on the respective sets of imaging parameter values, the plurality of sets of ultrasound images;
determines a set of ultrasound images from among the plurality of sets of ultrasound images that has a highest quality by determining the set of ultrasound images from among the plurality of sets of ultrasound images for which a view classifier has a highest confidence that the view classifier recognizes an anatomical region in the set of ultrasound images; and
based on the determined set of ultrasound images that has the highest quality, provides a prompt as to whether to configure the ultrasound system (700) to produce ultrasound images using a set of imaging parameter values with which the set of ultrasound images that has the highest quality was produced.

2. The ultrasound system (700) of claim 1, wherein the ultrasound system (700) is configured to produce the plurality of sets of ultrasound images based on detecting, by the processing device (702; 200), that the ultrasound system (700) has begun imaging a subject (406; 506) after not imaging the subject (406; 506) for a threshold period of time.

3. The ultrasound system (700) of claim 2, wherein the processing device (702; 200), while detecting that the ultrasound system (700) has begun imaging the subject (406; 506) after not imaging the subject (406; 506) for the threshold period of time, configures the ultrasound imaging device (706) with a low-power set of imaging parameter values that uses less power than the plurality of sets of imaging parameter values.

4. The ultrasound system (700) of claim 1, wherein the processing device (702; 200) , when determining the set of ultrasound images from among the plurality of sets of ultrasound images that has the highest quality, calculates at least one selected from a group consisting of:
an image sharpness metric for each of the plurality of sets of ultrasound images,
a pixel variation metric for each of the plurality of sets of ultrasound images,
a noise metric for each of the plurality of sets of ultrasound images,
a total variation metric for each of the plurality of sets of ultrasound images, and
a pixel intensity metric for each of the plurality of sets of ultrasound images.

5. The ultrasound system (700) of claim 1, wherein the processing device (702; 200) generates an instruction for a user to hold substantially stationary the ultrasound imaging device (706) while the ultrasound system (700) is producing the plurality of sets of ultrasound images.

6. The ultrasound system (700) of claim 1, wherein the plurality of sets of imaging parameter values comprise ultrasound imaging presets each optimized for imaging a particular anatomical region among a plurality of anatomical regions.

7. The ultrasound system (700) of claim 6,
wherein the plurality of anatomical regions comprise a plurality of anatomical regions imaged during a particular ultrasound imaging protocol, and
wherein the processing device (702; 200) receives an input from a user that the user will be performing the particular ultrasound imaging protocol.

8. The ultrasound system (700) of claim 1, wherein the plurality of sets of imaging parameter values comprise preferred sets of imaging parameter values associated with a user.

9. The ultrasound system (700) of claim 1, wherein configuring the ultrasound system (700) to produce the plurality of sets of ultrasound images comprises configuring the ultrasound system (700) to:
transmit a plurality of sets of ultrasound waves into a subject (406; 506) using the plurality of sets of imaging parameter values, wherein the plurality of sets of imaging parameter values relate to ultrasound transmission; and
generate each of the plurality of sets of ultrasound images from a different set of reflected ultrasound waves each corresponding to one of the plurality of sets of transmitted ultrasound waves.

10. The ultrasound system (700) of claim 1, wherein configuring the ultrasound system (700) to produce the plurality of sets of ultrasound images comprises configuring the ultrasound system (700) to:
transmit a single set of ultrasound waves into a subject (406; 506); and
generate each of the plurality of sets of ultrasound images from a single set of reflected ultrasound waves corresponding to the single set of transmitted ultrasound waves using the plurality of sets of imaging parameter values, wherein the plurality of sets of imaging parameter values relate to ultrasound image generation.

11. The ultrasound system (700) of claim 1, wherein the ultrasound system (700) is configured, when providing the prompt (414) as to whether to configure the ultrasound system (700) to produce ultrasound images using the set of imaging parameter values with which the set of ultrasound images that has the highest quality was produced, to provide a notification of an anatomical region (512; 612) for which the set of imaging parameter values is optimized.

12. A method for configuring an ultrasound system (700) to produce a plurality of sets of ultrasound images, the method comprising:
selecting, for a subsequent obtaining of each respective set of the plurality of sets of ultrasound images, a different respective set of a plurality of sets of imaging parameter values;
obtaining, using an ultrasound imaging device (706) of the ultrasound system (700), and based on the respective sets of imaging parameter values, the plurality of sets of ultrasound images;
determining, based on a view classifier having a highest confidence that the view classifier recognizes an anatomical region in a set of ultrasound images, the set of ultrasound images from among the plurality of sets of ultrasound images that has a highest quality; and
based on the determining the set of ultrasound images that has the highest quality,
providing a prompt as to whether to configure the ultrasound system to produce ultrasound images using the set of imaging parameter values with which the set of ultrasound images that has the highest quality was produced.

13. The method of claim 12, wherein determining the set of ultrasound images that has the highest quality comprises calculating at least one selected from a group consisting of:
an image sharpness metric for each of the plurality of sets of ultrasound images,
a pixel variation metric for each of the plurality of sets of ultrasound images,
a noise metric for each of the plurality of sets of ultrasound images,
a total variation metric for each of the plurality of sets of ultrasound images, and
a pixel intensity metric for each of the plurality of sets of ultrasound images.

14. A non-transitory computer-readable storage medium comprising instructions which, when executed by a processor of an ultrasound system (700) comprising an ultrasound imaging device (706), cause the processor to perform the method of claim 12 or claim 13.

## Patentansprüche

1. Ultraschallsystem (700) umfassend:
eine Ultraschall-Bildgebungsvorrichtung (706); und
eine Verarbeitungsvorrichtung (702; 200), die:
das Ultraschallsystem (700) dafür konfiguriert, eine Vielzahl von Sätzen von Ultraschallbildern zu erzeugen durch: für ein nachfolgendes Erhalten von jedem entsprechenden Satz der Vielzahl von Sätzen von Ultraschallbildern Auswählen eines anderen entsprechenden Satzes aus einer Vielzahl von Sätzen von Bildgebungsparameterwerten;
unter Verwendung der Ultraschall-Bildgebungsvorrichtung und auf der Grundlage der entsprechenden Sätze von Bildgebungsparameterwerten die Vielzahl von Sätzen von Ultraschallbildern erhält;
einen Satz von Ultraschallbildern aus der Vielzahl von Sätzen von Ultraschallbildern bestimmt, der eine höchste Qualität aufweist, durch Bestimmen des Satzes von Ultraschallbildern aus der Vielzahl von Sätzen von Ultraschallbildern, für den ein Ansichtsklassifikator einen höchste Konfidenz aufweist, dass der Ansichtsklassifikator einen anatomischen Bereich in dem Satz von Ultraschallbildern erkennt; und
auf der Grundlage des bestimmten Satzes von Ultraschallbildern, der die höchste Qualität aufweist, einen Anfrage bereitstellt, ob das Ultraschallsystem (700) zu konfigurieren ist, um Ultraschallbilder unter Verwendung eines Satzes von Bildgebungsparameterwerten zu erzeugen, mit denen der Satz von Ultraschallbildern mit der höchsten Qualität erzeugt worden ist.

2. Ultraschallsystem (700) nach Anspruch 1, wobei das Ultraschallsystem (700) dafür konfiguriert ist, die Vielzahl von Sätzen von Ultraschallbildern auf der Grundlage der Erfassung durch die Verarbeitungsvorrichtung (702; 200) zu erzeugen, dass das Ultraschallsystem (700) begonnen hat, Bilder von einem Subjekt (406; 506) aufzunehmen, nachdem es für einen Zeit-Schwellenwert keine Bilder von einem Subjekt (406; 506) aufgenommen hat.

3. Ultraschallsystem (700) nach Anspruch 2, wobei die Verarbeitungsvorrichtung (702; 200) bei Erfassen, dass das Ultraschallsystem (700) begonnen hat, Bilder von einem Subjekt (406; 506) aufzunehmen, nachdem es für den Zeit-Schwellenwert keine Bilder von einem Subjekt (406; 506) aufgenommen hat, die Ultraschall-Bildgebungsvorrichtung (706) mit einem leistungsarmen Satz von Bildgebungsparameterwerten konfiguriert, der weniger Leistung verbraucht als die Vielzahl von Sätzen von Bildgebungsparameterwerten.

4. Ultraschallsystem (700) nach Anspruch 1, wobei die Verarbeitungsvorrichtung (702; 200) bei der Bestimmung des Satzes von Ultraschallbildern aus der Vielzahl von Sätzen von Ultraschallbildern, der die höchste Qualität aufweist, wenigstens eines berechnet aus einer Gruppe bestehend aus:
einer Bildschärfemetrik für jeden aus der Vielzahl von Sätzen von Ultraschallbildern,
einer Pixelvariationsmetrik für jeden aus der Vielzahl von Sätzen von Ultraschallbildern,
einer Rauschmetrik für jeden aus der Vielzahl von Sätzen von Ultraschallbildern,
einer Gesamtvariationsmetrik für jeden aus der Vielzahl von Sätzen von Ultraschallbildern und
einer Pixelintensitätsmetrik für jeden aus der Vielzahl von Sätzen von Ultraschallbildern.

5. Ultraschallsystem (700) nach Anspruch 1, wobei die Verarbeitungsvorrichtung (702; 200) eine Anweisung für einen Benutzer erzeugt, die Ultraschallbildgebungsvorrichtung (706) im Wesentlichen stationär zu halten, während das Ultraschallsystem (700) die Vielzahl von Sätzen von Ultraschallbildern erzeugt.

6. Ultraschallsystem (700) nach Anspruch 1, wobei die Vielzahl von Sätzen von Bildgebungsparameterwerten Ultraschallbildgebungsvoreinstellungen umfasst, die jeweils für die Bildgebung eines bestimmten anatomischen Bereichs unter einer Vielzahl von anatomischen Bereichen optimiert sind.

7. Ultraschallsystem (700) nach Anspruch 6,
wobei die Vielzahl von anatomischen Bereichen eine Vielzahl von anatomischen Bereichen umfasst, die während eines bestimmten Ultraschallbildgebungsprotokolls abgebildet werden, und
wobei die Verarbeitungsvorrichtung (702; 200) eine Eingabe von einem Benutzer erhält, dass der Benutzer das bestimmte Ultraschallbildgebungsprotokoll durchführen wird.

8. Ultraschallsystem (700) nach Anspruch 1, wobei die Vielzahl von Sätzen von Bildgebungsparameterwerten bevorzugte Sätze von Bildgebungsparameterwerten umfasst, die mit einem Benutzer verbunden sind.

9. Ultraschallsystem (700) nach Anspruch 1, wobei Konfigurieren des Ultraschallsystems (700) zum Erzeugen der Vielzahl von Sätzen von Ultraschallbildern Konfigurieren des Ultraschallsystems (700) umfasst zum:
Senden einer Vielzahl von Sätzen von Ultraschallwellen in ein Subjekt (406; 506) unter Verwendung der Vielzahl von Sätzen von Bildgebungsparameterwerten, wobei die Vielzahl von Sätzen von Bildgebungsparameterwerten mit Ultraschallsenden verbunden ist; und
Erzeugen von jedem der Vielzahl von Sätzen von Ultraschallbildern aus einem anderen Satz von reflektierten Ultraschallwellen, die jeweils einem aus der Vielzahl von Sätzen von gesendeten Ultraschallwellen entsprechen.

10. Ultraschallsystem (700) nach Anspruch 1, wobei Konfigurieren des Ultraschallsystems (700) zum Erzeugen der Vielzahl von Sätzen von Ultraschallbildern Konfigurieren des Ultraschallsystems (700) umfasst zum:
Senden eines einzelnen Satzes von Ultraschallwellen in ein Subjekt (406; 506); und
Erzeugen von jedem der Vielzahl von Sätzen von Ultraschallbildern aus einem einzelnen Satz von reflektierten Ultraschallwellen, die dem einzelnen Satz von gesendeten Ultraschallwellen entsprechen, unter Verwendung der Vielzahl von Sätzen von Bildgebungsparameterwerten, wobei die Vielzahl von Sätzen von Bildgebungsparameterwerten mit Ultraschallbilderzeugung verbunden ist.

11. Ultraschallsystem (700) nach Anspruch 1, wobei das Ultraschallsystem (700) konfiguriert ist, bei der Bereitstellung der Anfrage (414), ob das Ultraschallsystem (700) zu konfigurieren ist, um Ultraschallbilder unter Verwendung des Satzes von Bildgebungsparameterwerten zu erzeugen, mit denen der Satz von Ultraschallbildern mit der höchsten Qualität erzeugt worden ist, eine Angabe eines anatomischen Bereichs (512; 612) bereitzustellen, für den der Satz von Bildgebungsparameterwerten optimiert ist.

12. Verfahren zum Konfigurieren eines Ultraschallsystems (700) zur Herstellung einer Vielzahl von Sätzen von Ultraschallbildern, wobei das Verfahren umfasst:
für ein nachfolgendes Erhalten von jedem entsprechenden Satz der Vielzahl von Sätzen von Ultraschallbildern, Auswählen eines anderen entsprechenden Satzes aus einer Vielzahl von Sätzen von Bildgebungsparameterwerten;
unter Verwendung einer Ultraschall-Bildgebungsvorrichtung (706) des Ultraschallsystems (700) und auf der Grundlage der entsprechenden Sätze von Bildgebungsparameterwerten, Erhalten der Vielzahl von Sätzen von Ultraschallbildern;
auf der Grundlage eines Ansichtklassifikators mit einer höchsten Konfidenz, dass der Ansichtsklassifikator einen anatomischen Bereich in dem Satz von Ultraschallbildern erkennt, Bestimmen des Satzes von Ultraschallbildern aus der Vielzahl von Sätzen von Ultraschallbildern, der eine höchste Qualität aufweist; und
auf der Grundlage der Bestimmung des Satzes von Ultraschallbildern, der die höchste Qualität aufweist, Bereitstellen einer Anfrage, ob das Ultraschallsystem zu konfigurieren ist, um Ultraschallbilder unter Verwendung des Satzes von Bildgebungsparameterwerten zu erzeugen, mit denen der Satz von Ultraschallbildern mit der höchsten Qualität erzeugt worden ist.

13. Verfahren nach Anspruch 12, wobei Bestimmen des Satzes von Ultraschallbildern, der die höchste Qualität aufweist, Berechnen von wenigstens einem aus einer Gruppe bestehend aus:
einer Bildschärfemetrik für jeden aus der Vielzahl von Sätzen von Ultraschallbildern,
einer Pixelvariationsmetrik für jeden aus der Vielzahl von Sätzen von Ultraschallbildern,
einer Rauschmetrik für jeden aus der Vielzahl von Sätzen von Ultraschallbildern,
einer Gesamtvariationsmetrik für jeden aus der Vielzahl von Sätzen von Ultraschallbildern und
einer Pixelintensitätsmetrik für jeden aus der Vielzahl von Sätzen von Ultraschallbildern,
umfasst.

14. Nichtflüchtiges computerlesbares Speichermedium, das Anweisungen umfasst, die bei Ausführung durch einen Prozessor eines Ultraschallsystems (700), das eine Ultraschall-Bildgebungsvorrichtung (706) umfasst, den Prozessor veranlasst, das Verfahren nach Anspruch 12 oder Anspruch 13 durchzuführen.

## Revendications

1. Un système à ultrasons (700) comprenant :
un dispositif d'imagerie par ultrasons (706) ;
et un dispositif de traitement (702 ; 200) qui :
configure le système à ultrasons (700) pour produire une pluralité d'ensembles d'images échographiques en sélectionnant, pour l'obtention ultérieure de chaque ensemble respectif de la pluralité d'ensembles d'images échographiques, un ensemble distinct respectif d'une pluralité d'ensembles de valeurs de paramètres d'imagerie ;
obtient, en utilisant le dispositif d'imagerie par ultrasons, et sur la base des ensembles respectifs de valeurs de paramètres d'imagerie, la pluralité d'ensembles d'images échographiques ;
détermine, parmi la pluralité d'ensembles d'images échographiques, l'ensemble d'images échographiques ayant la qualité la plus élevée en déterminant l'ensemble d'images échographiques parmi ladite pluralité pour lequel un classifieur de vues présente la confiance la plus élevée que le classifieur de vues reconnaît une région anatomique dans l'ensemble d'images échographiques ; et
sur la base de l'ensemble d'images échographiques ainsi déterminé qui présente la qualité la plus élevée, fournit une invite demandant s'il faut configurer le système à ultrasons (700) pour produire des images échographiques en utilisant un ensemble de valeurs de paramètres d'imagerie avec lequel l'ensemble d'images échographiques présentant la qualité la plus élevée a été produit.

2. Le système à ultrasons (700) selon la revendication 1, dans lequel le système à ultrasons (700) est configuré pour produire la pluralité d'ensembles d'images échographiques sur la base de la détection, par le dispositif de traitement (702 ; 200), que le système à ultrasons (700) a commencé à imager un sujet (406 ; 506) après ne pas avoir imagé le sujet (406 ; 506) pendant une période seuil.

3. Le système à ultrasons (700) selon la revendication 2, dans lequel le dispositif de traitement (702 ; 200), tout en détectant que le système à ultrasons (700) a commencé à imager le sujet (406 ; 506) après ne pas avoir imagé le sujet (406 ; 506) pendant la période seuil, configure le dispositif d'imagerie par ultrasons (706) avec un ensemble de valeurs de paramètres d'imagerie à faible énergie qui utilise moins d'énergie que la pluralité d'ensembles de valeurs de paramètres d'imagerie.

4. Le système à ultrasons (700) selon la revendication 1, dans lequel le dispositif de traitement (702 ; 200), lorsqu'il détermine l'ensemble d'images échographiques parmi la pluralité d'ensembles d'images échographiques qui présente la qualité la plus élevée, calcule au moins un élément sélectionné dans un groupe consistant en :
une métrique d'acuité d'image pour chacun des ensembles de la pluralité d'ensembles d'images échographiques,
une métrique de variation de pixels pour chacun des ensembles de la pluralité d'ensembles d'images échographiques,
une métrique de bruit pour chacun des ensembles de la pluralité d'ensembles d'images échographiques,
une métrique de variation totale pour chacun des ensembles de la pluralité d'ensembles d'images échographiques, et
une métrique d'intensité de pixels pour chacun des ensembles de la pluralité d'ensembles d'images échographiques.

5. Le système à ultrasons (700) selon la revendication 1, dans lequel le dispositif de traitement (702 ; 200) génère une instruction à l'utilisateur de maintenir substantiellement immobile le dispositif d'imagerie par ultrasons (706) pendant que le système à ultrasons (700) est en train de produire la pluralité d'ensembles d'images échographiques.

6. Le système à ultrasons (700) selon la revendication 1, dans lequel la pluralité d'ensembles de valeurs de paramètres d'imagerie comprend des préréglages d'imagerie par ultrasons chacun optimisé pour l'imagerie d'une région anatomique particulière parmi une pluralité de régions anatomiques.

7. Le système à ultrasons (700) selon la revendication 6,
dans lequel la pluralité de régions anatomiques comprend une pluralité de régions anatomiques imagées lors d'un protocole d'imagerie par ultrasons particulier, et
dans lequel le dispositif de traitement (702 ; 200) reçoit une saisie de la part d'un utilisateur indiquant que celui-ci va réaliser le protocole d'imagerie par ultrasons particulier.

8. Le système à ultrasons (700) selon la revendication 1, dans lequel la pluralité d'ensembles de valeurs de paramètres d'imagerie comprend des ensembles préférés de valeurs de paramètres d'imagerie associés à un utilisateur.

9. Le système à ultrasons (700) selon la revendication 1, dans lequel la configuration du système à ultrasons (700) pour produire la pluralité d'ensembles d'images échographiques comprend la configuration du système à ultrasons (700) pour :
la transmission d'une pluralité d'ensembles d'ondes ultrasonores dans un sujet (406 ; 506) en utilisant la pluralité d'ensembles de valeurs de paramètres d'imagerie, la pluralité d'ensembles de valeurs de paramètres d'imagerie se rapportant à la transmission d'ultrasons ; et
la génération de chacun des ensembles d'images échographiques de la pluralité à partir d'un ensemble distinct d'ondes ultrasonores réfléchies, chaque ensemble correspondant à l'un des ensembles d'ondes ultrasonores transmises.

10. Le système à ultrasons (700) selon la revendication 1, dans lequel la configuration du système à ultrasons (700) pour produire la pluralité d'ensembles d'images échographiques comprend la configuration du système à ultrasons (700) pour :
la transmission d'un seul ensemble d'ondes ultrasonores dans un sujet (406 ; 506) ; et
la génération de chacun des ensembles d'images échographiques de la pluralité à partir d'un seul ensemble d'ondes ultrasonores réfléchies correspondant au seul ensemble d'ondes ultrasonores transmises, en utilisant la pluralité d'ensembles de valeurs de paramètres d'imagerie, la pluralité d'ensembles de valeurs de paramètres d'imagerie se rapportant à la génération d'images échographiques.

11. Le système à ultrasons (700) selon la revendication 1, dans lequel le système à ultrasons (700) est configuré, lors de la fourniture de l'invite (414) demandant s'il faut configurer le système à ultrasons (700) pour produire des images échographiques en utilisant l'ensemble de valeurs de paramètres d'imagerie avec lequel l'ensemble d'images échographiques présentant la qualité la plus élevée a été produit, à fournir une notification d'une région anatomique (512 ; 612) pour laquelle l'ensemble de valeurs de paramètres d'imagerie est optimisé.

12. Un procédé de configuration d'un système à ultrasons (700) pour produire une pluralité d'ensembles d'images échographiques, le procédé comprenant :
la sélection, pour l'obtention ultérieure de chaque ensemble respectif de la pluralité d'ensembles d'images échographiques, d'un ensemble distinct respectif d'une pluralité d'ensembles de valeurs de paramètres d'imagerie ;
l'obtention, en utilisant un dispositif d'imagerie par ultrasons (706) du système à ultrasons (700), et sur la base des ensembles respectifs de valeurs de paramètres d'imagerie, de la pluralité d'ensembles d'images échographiques ;
la détermination, sur la base d'un classifieur de vues présentant la confiance la plus élevée que ledit classifieur reconnaît une région anatomique dans un ensemble d'images échographiques, de l'ensemble d'images échographiques parmi la pluralité d'ensembles d'images échographiques qui présente la qualité la plus élevée ; et
sur la base de la détermination de l'ensemble d'images échographiques qui présente la qualité la plus élevée, la fourniture d'une invite demandant s'il faut configurer le système à ultrasons pour produire des images échographiques en utilisant l'ensemble de valeurs de paramètres d'imagerie avec lequel l'ensemble d'images échographiques présentant la qualité la plus élevée a été produit.

13. Le procédé selon la revendication 12, dans lequel la détermination de l'ensemble d'images échographiques présentant la qualité la plus élevée comprend le calcul d'au moins un élément sélectionné dans un groupe consistant en :
une métrique d'acuité d'image pour chacun des ensembles de la pluralité d'ensembles d'images échographiques,
une métrique de variation de pixels pour chacun des ensembles de la pluralité d'ensembles d'images échographiques,
une métrique de bruit pour chacun des ensembles de la pluralité d'ensembles d'images échographiques,
une métrique de variation totale pour chacun des ensembles de la pluralité d'ensembles d'images échographiques, et
une métrique d'intensité de pixels pour chacun des ensembles de la pluralité d'ensembles d'images échographiques.

14. Un support de stockage non transitoire lisible par ordinateur comprenant des instructions qui, lorsqu'elles sont exécutées par un processeur d'un système à ultrasons (700) comprenant un dispositif d'imagerie par ultrasons (706), font exécuter au processeur le procédé selon la revendication 12 ou la revendication 13.
